(19) **Europäisches Patentamt / European Patent Office / Office européen des brevets**

(11) **EP 3 787 582 B1**

(12) **EUROPEAN PATENT SPECIFICATION**

(45) Date of publication and mention
of the grant of the patent:
**02.07.2025 Bulletin 2025/27**

(21) Application number: **19724681.2**

(22) Date of filing: **03.05.2019**

(51) International Patent Classification (IPC):
**A61F 13/15** *(2006.01)*      **G01N 27/04** *(2006.01)*

(52) Cooperative Patent Classification (CPC):
**A61F 13/15772; G01N 27/048;** A61F 2013/1578;
A61F 2013/422

(86) International application number:
**PCT/US2019/030684**

(87) International publication number:
**WO 2019/213586 (07.11.2019 Gazette 2019/45)**

(54) **DETERMINING DIAPER LOADING USING COLOR DETECTION OR ACTIVITY STATE**

BESTIMMUNG DER WINDELBELADUNG UNTER VERWENDUNG MITTELS FARBDETEKTION ODER AKTIVITÄTSZUSTAND

DÉTERMINATION DU CHARGEMENT D'UNE COUCHE À L'AIDE D'UNE DÉTECTION DE COULEUR OU D'UN ÉTAT D'ACTIVITÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.05.2018   US 201815971306**
**12.04.2019   US 201916383337**

(43) Date of publication of application:
**10.03.2021   Bulletin 2021/10**

(73) Proprietor: **Verily Life Sciences LLC**
**Dallas, TX 75019 (US)**

(72) Inventors:
• **SCHIFFER, Brian**
**San Francisco, California 94080 (US)**
• **PARLIKAR, Tushar**
**San Francisco, California 94080 (US)**
• **GUILARDI, Brian Ward**
**San Francisco, California 94080 (US)**

(74) Representative: **Mewburn Ellis LLP**
**Aurora Building**
**Counterslip**
**Bristol BS1 6BX (GB)**

(56) References cited:
**EP-A1- 2 832 323     WO-A1-2007/128038**

## Description

### FIELD

The present application generally relates to a computer-implemented method and system for determining a volume of bodily exudate or excretion present in an absorbent article (e.g. a wearable absorbent article) and more specifically relates to determining a volume of urine in a diaper based on a changing color of a color changing indicator in the diaper and/or a detected activity of the wearer of the diaper.

### BACKGROUND

Existing solutions exist for determining a level of bodily exudate or excretion in an absorbent article are inadequate. For example, some existing solutions rely on use temperature or humidity sensors alone, which can lead to inaccurate measurements. For example, a humidity sensor may be located too far away from bodily exudate to detect a sudden increase in humidity. Or a temperature sensor may indicate an elevated temperature, but the location of sensing may not be representative of the temperature of the absorbent article overall.

Finally, other solutions for detecting a level of bodily exudate or excretion present are prone to erroneous measurements due to movement of the wearer. For example, a sensor measurement can include noise or error caused by either motion of the sensor relative to absorbent article or motion of the absorbent article itself (e.g., due to the wearer of the absorbent article moving).

Hence, new solutions are needed for at least the reasons described above.

WO2007128038 discloses a moisture monitoring system for monitoring wetness in one or more absorbent articles.

### SUMMARY

Aspects of the invention are set out in the appended claims.

### BRIEF DESCRIPTION OF THE DRAWINGS

Features, embodiments, and advantages of the present disclosure are better understood when the following Detailed Description is read with reference to the accompanying drawings.

Figure 1 depicts a block diagram of an example of an infant sensing system, according to certain aspects of the present disclosure.

Figure 2 depicts a block diagram of an example of a color detection system, according to certain aspects of the present disclosure.

Figure 3 depicts an absorbent article with color changing indicator and sensing device, according to certain aspects of the present disclosure.

Figure 4 includes Figures 4A and 4B, according to certain aspects of the present disclosure. Figure 4A represents a top side view of an example of a sensor layout for sensor package 400. Figure 4B represents a bottom side view of an example of a sensor layout for sensor package 400.

Figure 5 depicts an example color detector cell configuration, according to certain aspects of the present disclosure.

Figure 6 is a flowchart that describes a method of detecting of color, according to certain aspects of the present disclosure.

Figure 7 is a flowchart of an exemplary method used to determine activity from a movement sensor, according to certain aspects of the present disclosure.

Figure 8 is a flowchart of an exemplary method used to determine activity from a movement sensor by using a predictive model, according to certain aspects of the present disclosure.

Figure 9 is a flowchart that describes a method of detecting a volume of bodily exudate in an absorbent article, according to certain aspects of the present disclosure.

Figure 10 is a diagram depicting an example computing system for performing functions related to color detection and detection of bodily exudate, according to some aspects of the present disclosure.

### DETAILED DESCRIPTION

Aspects described herein provide solutions for accurately determining a volume of bodily exudate or excretion (e.g., urine or feces) in an absorbent article by using a color detection system in conjunction with an activity classification system. The absorbent article is preferably a wearable structure, e.g. an infant diaper, or the like. More specifically, an example color detection system may use a pulsed light source to accurately detect the color of an object such as a color changing indicator in an absorbent article (e.g., a diaper). The color detection system may compensate for or adapt its operation to minimize the effects of ambient light, so that the system can be used even in the presence of ambient light. An activity classification system can determine an activity state of an infant wearing the absorbent article by analyzing measurements obtained from an inertial sensor attached to the absorbent article.

More specifically, an example color detection system includes one or more light sources such as LEDs, one or more photodetectors configured to detect light, and an electronic circuit or device such as a photometric front end or a general purpose processor configurable to receive information about detected color, filter out a contribution of the ambient light, and output a signal indicative of the detected color, e.g. as diaper condition information, for use in determining or controlling further operations. A color detection system is attached to an infant's diaper and oriented to allow the color detection system to shine light on a portion of the diaper containing a color changing indicator. A color changing indicator can change color, for example, based on the presence or absence of a substance to be detected (e.g. bodily exudate or excretion in examples herein).

An example activity classification system receives measurements from a movement sensor such as an accelerometer or gyroscope. The sensor is placed on the wearer, e.g., secured to the absorbent article, for example by pinning to a diaper. By using a predictive model or state machine, the activity classification system determines whether the wearer is awake or asleep. Whether the wearer is awake or asleep, in conjunction with other data such as the diaper condition information discussed above, improves the accuracy and reliability of detecting a presence or volume of bodily exudate such as urine. As such, the systems disclosed herein provide advantages over systems that rely solely on detection of a color of a color changing indicator in a diaper or another sensor, thereby facilitating correction or avoidance of errors caused by movement of the sensor or the wearer of the sensor.

These illustrative examples are given to introduce the reader to the general subject matter discussed herein and the disclosure is not limited to this example. The following sections describe various additional non-limiting examples and examples of color sensing using pulsed light.

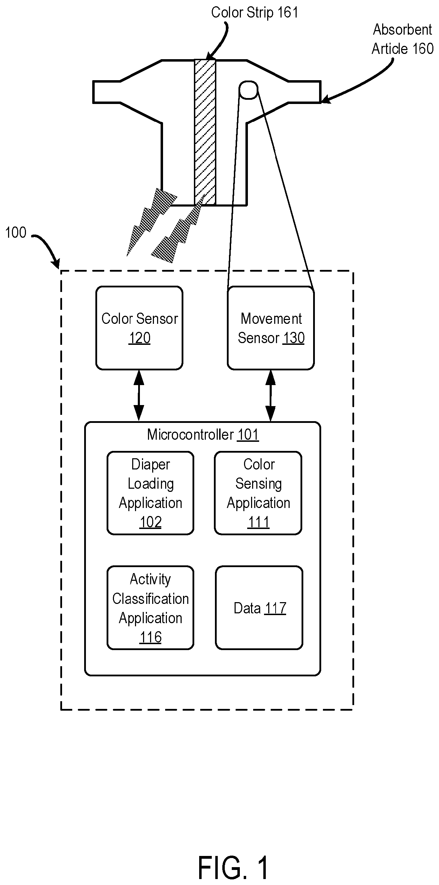

Figure 1 depicts a block diagram of an example of an infant sensing system, according to certain aspects of the present disclosure. Figure 1 depicts infant sensing system 100, which includes color sensor 120, movement sensor 130, and microcontroller 101. Microcontroller 101 includes diaper loading application 102, color sensing application 111, activity classification application 116, and data 117. Data 117 can include demographic data (sex, age, weight, etc.), data about the material qualities of an absorbent article, an activity state of an infant, and so on. Data 117 can be input via a user interface, e.g., from a caregiver, or downloaded from an external device.

In an example, diaper loading application 102 operates in conjunction with color sensing application 111 and activity classification application 116 to determine a volume of bodily exudate such as urine in absorbent article 160. As explained further herein, absorbent article 160 can include a color strip 161 that responds to a presence or amount of urine. In one example, color sensing application 111 operates to determine a change in color in color strip 161. In another example, color sensing application 111 can also be used to detect the color (e.g. output a signal indicative of detected color as opposed to a detected change in color) of any other object such as a color changing indicator that changes color based on the presence of another chemical. The color sensing application may be further configured to determine a presence of urine (or other chemical) in absorbent article 160 using the detected color or detected change in color. The color sensing application may output a signal indicative of the presence of urine. The signal may be binary, or may be a graded score based on the detected color of magnitude of color change.

Activity classification application 116 can determine a state of an infant wearing absorbent article 160. Movement sensor 130, which can be an accelerometer, gyroscope, or other sensor type, can be placed or adhered to absorbent article 160. Activity classification application 116 can determine a state of an infant such as whether the infant is asleep, awake, resting, and so on.

In an example, the movement sensor 130 is attached to an infant's clothing or absorbent article 160. The movement sensor 130, which can include an accelerometer or a gyroscope, provides measurements. Activity classification application 116 receives the measurements from the movement sensor 130 and uses a predictive model such as a machine learning model, state-flow-model, or algorithm to determine activities performed by the infant wearing the movement sensor. The predictive model is trained to determine, based on the infant's movement, an activity that the infant is performing such as sleep or sitting up. The predictive model can be trained by data gathered for an individual or group of individuals.

For example, the predictive model can receive training data that represents a particular individual. The training data can include the individual's movements as determined by movement sensor 130 and an associated ground truth (correct activity, as designated by a training label). Thus, upon receiving new data from movement sensor 130, the trained predictive model predicts an activity. In another example, the predictive model can be trained based on training data gathered from a group of individuals, which need not include the individual whose activity is later predicted. Therefore, the predictive model can learn from the recorded behavior of a particular individual or a group of individuals and use the learned behavior to predict an activity of the individual or another individual.

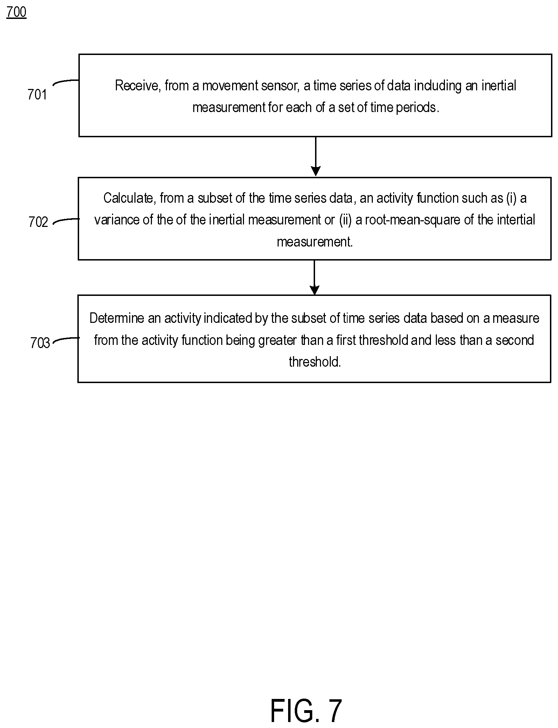

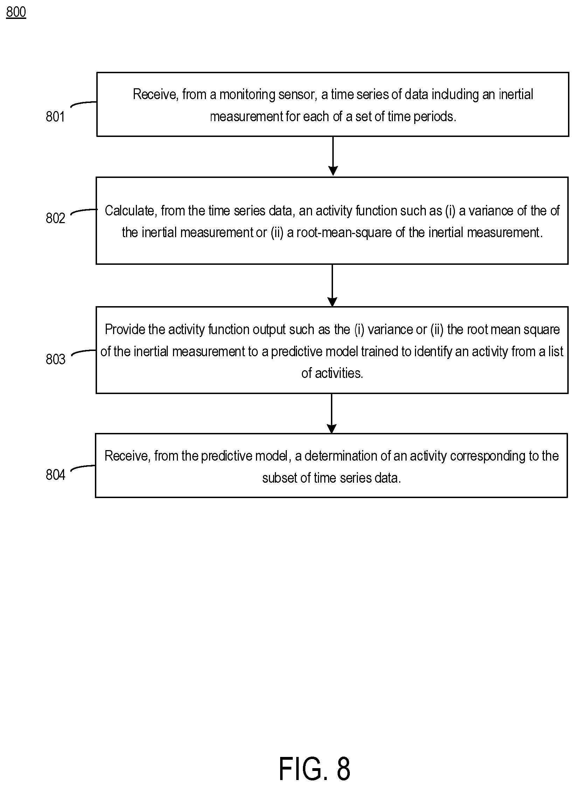

The activity classification system can then output a signal indicative of predicted activity information of the infant, for example that the baby is in a deep sleep, for use in determining or controlling further operations. Examples of suitable processes for activity classification are described with respect to Figures 7 and 8.

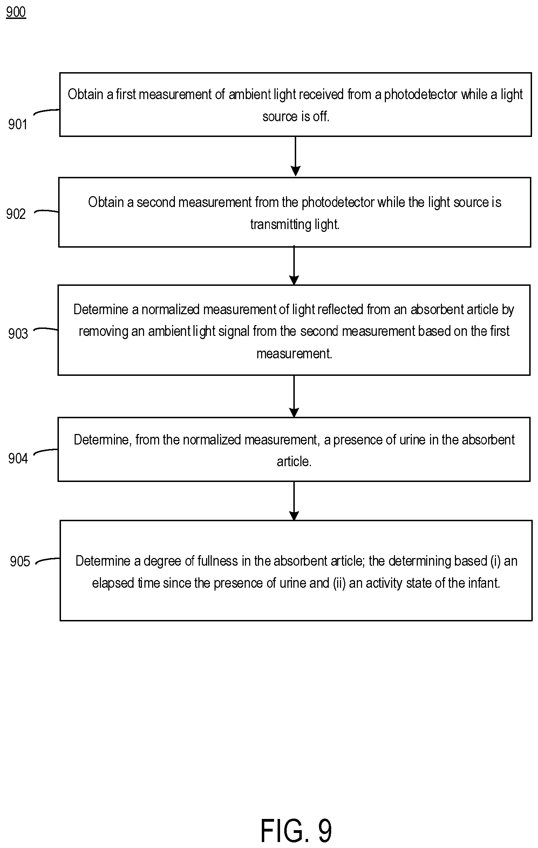

Using the diaper condition information, which may include an indication of a presence of urine from color sensing application 111, and the predicted activity information, which may include a predicted activity state from the activity classification application 116, the diaper loading application 102 can determine a volume of urine present in absorbent article 160. A volume of urine can be referred to as a diaper load. In the invention, statistical approaches are used. Different factors can be used such as an elapsed time since a first urination event, a diaper size, or the state of an infant. An example of a process used to determine a volume of urine in an absorbent article is described with respect to FIG. 9.

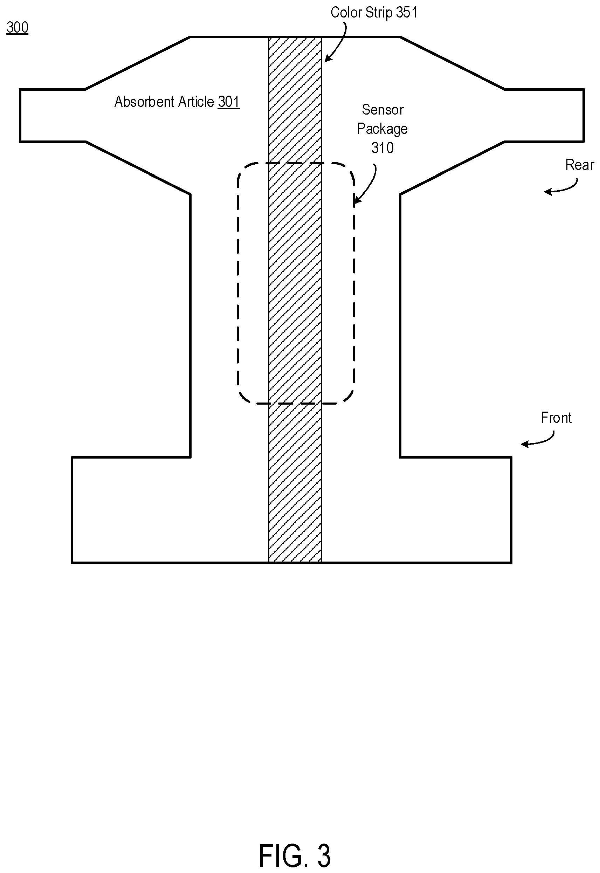

Infant sensing system 100 can be implemented as an accessory for attachment to a wearable article, e.g. on a slim material such as plastic or flexible substrate. For example, infant sensing system 100 can be 1-2 centimeters wide and 2-5 millimeters thick. Infant sensing system 100 can be made sufficiently small and thin to be placed in an absorbent article such as a diaper, as discussed with respect to Figure 3. In an example, infant sensing system 100 can be placed in a diaper that includes a color changing indicator such that a light source and a photodetector are aligned with the color changing indicator.

In an example, the infant sensing system 100 may be distributed between an accessory that is secured to the absorbent article, and an external device. For example, operations performed by microcontroller 110 can be performed by the external device. The accessory may thus include a communication module, e.g. transceiver or the like, for communication with the external device. Examples include a monitor connected by a local radio connection (e.g., WiFi or Bluetooth) and a remote server that connected to microcontroller 101 via the internet. For example, diaper loading application 102, color sensing application 111, and/or activity classification application 116 can delegate some or all of the operations described herein to an external device. Advantages include, but are not limited to, reducing battery life of infant sensing system 100, or improved performance due to availability of additional data sets, e.g., training data, or processing power on the external device.

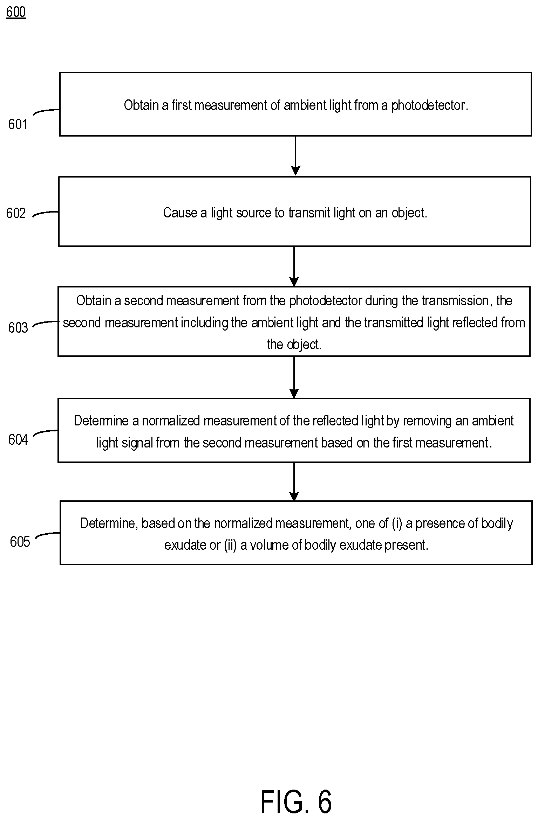

Any or all of the processes described herein, for example those discussed with respect to Figures 6-9 can be performed by the external device. For example, microcontroller 101 transmits data such as measured color from color sensor 120 or movement data from movement sensor 130 to the external device. In turn, the external device processes the operations to determine a significance of the detected color or movements.

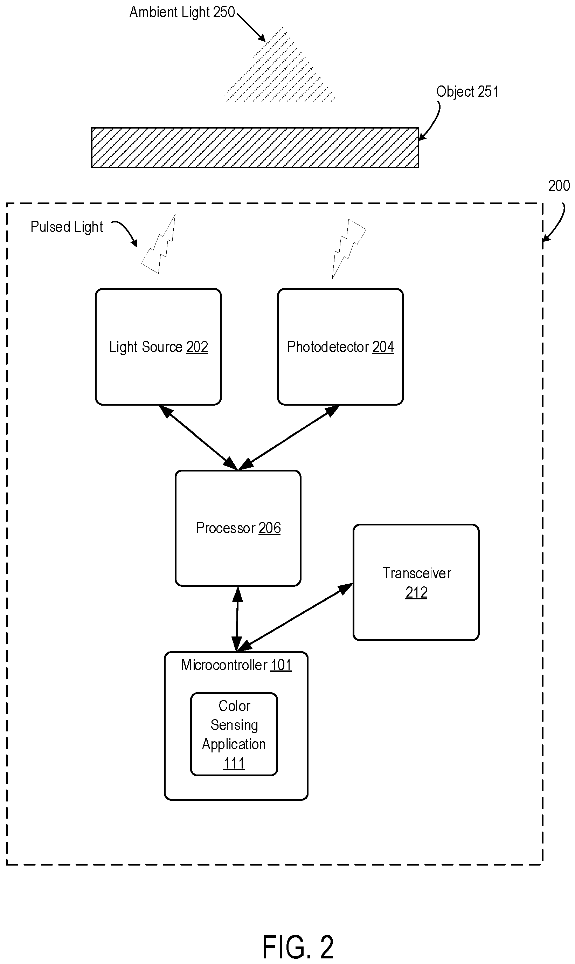

Figure 2 depicts a block diagram of an example of a color sensing system, according to certain aspects of the present disclosure. Figure 2 includes color sensing system 200, which includes light source 202, photodetector 204, processor 206, and microcontroller 101. Microcontroller 101 can implement the functionality of color sensing system 200, infant sensing system 100, or both. Further, for example purposes, microcontroller 101 is depicted in color sensing system 200, but a different microcontroller, microprocessor, or other processor can be used. In an aspect, only one of the processor 206 and the microcontroller 101 is present.

Color sensing system 200 can be configured to measure a color of an object 251 (e.g., an absorbent article or a color strip in an absorbent article) to determine a loading of an absorbent article. The system may obtain a measurement of color that is independent of the presence of ambient light, e.g. by measuring the ambient and compensating for it, or by adapting (e.g. tuning) the light source to reduce or minimize the effect of the ambient light on the detected signal.

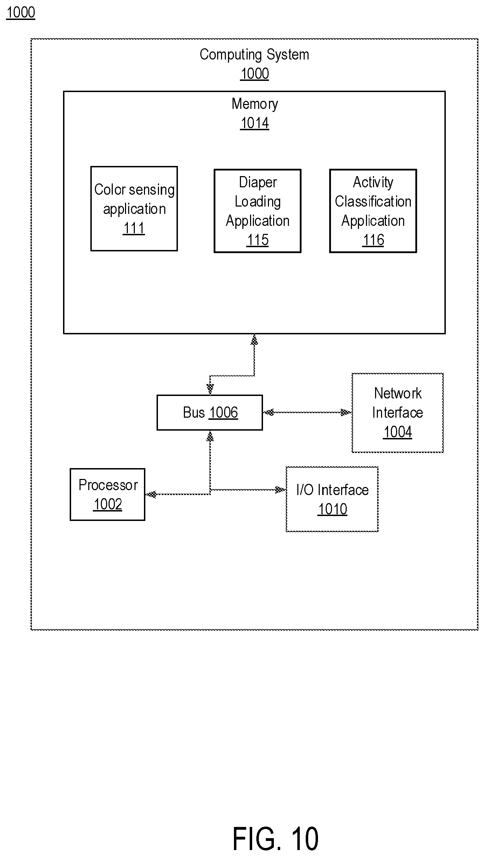

Color sensing system 200 also includes a microcontroller 101. Microcontroller 101 can be any controller, processor, application specific integrated circuit or other processing device. An example of a computing device is shown in Figure 10. Microcontroller 101101 can execute color sensing application 111 as well as other processor-executable instructions to perform aspects of the present disclosure.

The functions of microcontroller 101 can be implemented by processor 206 or vice versa. Microcontroller 101 can store data 117, which can include a state of an infant, demographic information about an infant, information about a particular absorbent article worn by an infant, and so forth.

Ambient light 250 can be any kind of light present in an environment that is not generated by light source 202, which can include light from natural sources, e.g., sunlight, or artificial light such as light created via incandescent light sources, halogen light sources, light emitting diode ("LED") light sources, fluorescent light sources, laser sources, etc. Even though ambient light can have different color spectra depending on the ambient light source(s) present, infant sensing system 100 can electronically remove the contribution of such ambient light to light detected by the photodetector and accurately detect the color of object 251 based on reflected light from the light source 202.

Light source 202 includes one or more light sources operable to shine light on object 251. The light sources can be any suitable artificial light source according to this disclosure, including LEDs, incandescent light sources, or other light sources. Multiple discrete light sources can be implemented individually or via an integrated package that combines multiple individual light sources into a single light source.

Light from light source 202 can be generated at one or more specific wavelengths, or can encompass multiple wavelengths. In an example, light source 202 has three sources of light: red light at wavelength 623 nanometers ("nm"), green light at wavelength 523 nm, and blue light at 455 nm wavelength. Other wavelengths of light may be employed according to different examples, depending on the application, the expected color range of a target object or color changing indicator such as a strip of litmus paper, expected ambient light spectra, or any other suitable factor. In some examples, the light source may be tunable to allow selection of a wavelength or wavelengths of light having a small contribution to the ambient light. For example, if ambient light detected by the photodetector indicates a local or global minimum magnitude at a first wavelength, the infant sensing system 100 can tune the light source 202 to emit light substantially at the first wavelength.

In this example, the color detection system pulses the light emitted by light source 202 by activating for a short duration, e.g., 1-5 microseconds to 500 milliseconds, called a "pulse width," and then deactivating the light source. Any suitable pulse width may be employed for a particular application. Light source 202 can create a separate pulse for red, blue, and green, and output the corresponding values. For example, a pulse width of 5 microseconds may be advantageous to detect a color of a color changing indicator. Short pulse widths enable the infant sensing system 100 to pulse and detect different colors of light, e.g., red, green, and blue, in quick succession of each other.

The use of pulsed light enables color sensing system 200 to disambiguate the type of light reflected by the object. Specifically, color sensing system 200 can detect and filter the ambient light from detected light that includes light pulsed from the light source 202. In some examples, the color sensing system 200 can pulse the light source 202 at regular intervals, e.g., every ten minutes, or in response to an event, such as a user pressing a button on the color detection system or a humidity sensor detecting a humidity level exceeding a threshold. Additionally, the use of pulsed light as compared to constant light can lower the power consumption of color sensing system 200, thereby increasing the amount of time that the color sensing system 200 can operate from a battery.

When the light source 202 is pulsed, the detected light at photodetector 204 may be a combination of ambient light 250 and light from the pulsed light source 202 reflected from the object 251. When the light source 202 is inactive, the light detected by the photodetector 204 is ambient light. By pulsing the light source 202, color sensing system 200 is able to first obtain baseline information about the ambient light spectrum to enable the color detection system to filter light received when the light source 202 is active. Pulsing also allows the infant sensing system 100 to save power by deactivating the light source 202 when a color measurement is not being taken.

Photodetector 204 receives a light, including light reflected from the object 251, whether ambient light or light emitted by the light source 202, and generates sensor signals based on that received light. Photodetector 204 can be any device that can detect and measure light such as a photodiode, phototransistor, complementary metal-oxide-semiconductor (CMOS) image sensor, charge-coupled device (CCD) sensor, or a photo-resistor.

Photodetector 204 can detect a wide spectrum of light and output information that indicates the detected light. For example, photodetector 204 can create an electrical output that is proportional to the wavelength of the received light. Photodetector 204 can provide three outputs of an RGB triplet, e.g., a value that corresponds to red, another value for green, and another value for blue.

More specifically, the values of the triplet correspond to the amplitude of light at a range of wavelengths corresponding to a particular color. Therefore, a first value is proportional to an amplitude of red in the received light, a second value is proportional to an amplitude of green in the received light, and a third value is proportional to an amplitude of blue in the received light.

In an aspect, a photodetector 204 can be an array of individual photodetectors. Each photodetector can be configured to measure a color of light. For example, one photodetector measures red, a second photodetector measures blue, and a third photodetector measures green.

Processor 206 is an electronic circuit or device such as a general-purpose processor. Processor 206 can operate in the analog domain, digital domain, or both. Processor 206 can discern the true color of the object 251 independent of any ambient light. Processor 206 receives a first output from photodetector 204 that represents the ambient light, for example, an output gathered when the light source 202 is off. Processor 206 receives a second output from photodetector 204 when the light source 202 is pulsed. Processor 206 discerns a difference between the first output and the second output and thereby isolates the color of the object, specifically the color of the reflected light on the object from the pulsed light.

In an aspect, processor 206 receives a level indicating an intensity of broad spectrum light that represents the ambient light, i.e., the point in time that the light source 202 is off, and a level indicating the intensity of for a second point in time at which one of the three colors red, blue, and green, is pulsed. Processor 206 can then disambiguate the contribution of the single pulsed color from the ambient light by comparing the intensity of the ambient light and the intensity with the single pulsed color.

Processor 206 receives a first set red, green, and blue levels from photodetector 204 for a point in time that the light source 202 is off and a second set of red, green, and blue levels from a second point in time that the light source 202 is pulsed. Processor 206 calculates a difference between the level of red between the first and second points in time, thereby calculating a contribution of red, green, and blue levels from the pulsed light.

Processor 206 may be a specialized photometric front end such as Analog Devices® ADPD105, ADPD106, or ADPD107. Processor 206 may be configured to activate light source 202 and measure a signal received by photodetector 204. For example, processor 206 can receive an analog input from photodetector 204, convert the analog input to a digital output by using a analog-to-digital converter (ADC), then store a numerical value indicating the detected color in an internal memory for later comparison with another value.

In this manner, processor 206 may be configured to disambiguate the contribution of the ambient light 250 in the analog domain and output an analog signal or digital value indicative of the color of object 251. For example, the processor 206 can provide an output, such as an RGB triplet value representing the color of object 251.

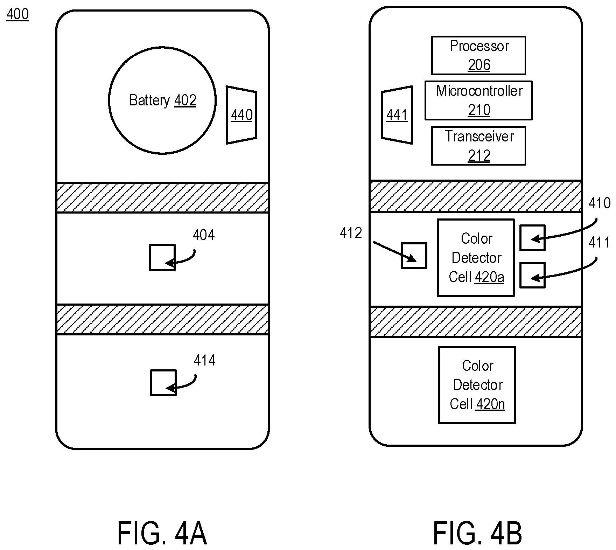

In an aspect, processor 206 can have multiple detection channels, each corresponding to a pair that of a light source 202 and a photodetector 204. As described further with respect to Figures 4A and 4B, each channel can be dedicated to a specific light source-photodetector pair, or a "cell." Each cell can be physically separated so that the processor 206 may measure color in multiple places. Processor 206 can also pulse the light from a particular cell differently from a light from another cell.

Color sensing application 111 can provide additional functionality such as calibration or white balancing for the signal received from light source 202. For example, microcontroller 101 receives a digital input indicating the color of the received light from processor 206. The digital input can include red, green, and blue levels. Color sensing application 111 can convert the red, green, and blue levels to hue, saturation, and lightness/value and perform calculations on the hue, saturation, and lightness/value.

Color sensing application 111 may also calibrate the received color value. For example, color sensing application 111 can retrieve known values such as the detected values when a known color, e.g. represented by a white or gray card or object that is presented to photodetector 204. Color sensing application 111 can adjust the received red, blue, and green levels according to the known calibration values.

In an aspect, microcontroller 101 may be connected to a transceiver 212. Transceiver 212 may communicate according to any suitable wireless protocol, such as Bluetooth, WiFi, near-field communication, etc. Using transceiver 212, microcontroller 101 may transmit the color of the object 251 or, if detecting bodily exudate in an absorbent article, notify an external device that an absorbent article has been soiled. Microcontroller 101 may transmit information to a remote device, such as a smartphone, smartwatch, or other wearable device, or a remote computer, such as a server, e.g., a cloud-based server, for further processing and analysis.

Microcontroller 101 can, via the transceiver 212, transmit the detected color from processor 206 to a remote server, which can map values that represent an expected reflected color from an object to a predicted volume of bodily exudate present in an absorbent article. Such a mapping can be accomplished via a table. For example, a table can contain a mapping between a Red-Blue-Green (RGB) triplet or range of triplets to a predicted volume of bodily exudate.

Object 251 can be a color changing indicator or other material that changes color based on the presence of a chemical. In an aspect, a color changing indicator can dissolve in the presence of a liquid such as urine. Accordingly, infant sensing system 100 can detect a change in color, an appearance of color, or a disappearance of color.

For example and as discussed further with respect to Figures 3-6, in one application, infant sensing system 100 is used to measure the presence of bodily exudate by reading a color changing indicator that changes color based on a presence or volume of a liquid. Exemplary color changing indicators include a pH strip or litmus paper strip that changes color based on detected pH level. Color sensing system 200 pulses light onto the color changing indicator and determines the amount of the pulsed light that is reflected.

More specifically, microcontroller 101 is programmed with data points from one or more wavelength-absorbance curves that correspond to different levels of acidity or pH level. By matching an absorbance level of a particular wavelength of light to a particular level of acidity, microcontroller 101 can determine a volume of a particular liquid, e.g., bodily exudate, or a specific pH level. For example, for a wavelength of light of 440 nm, if the measured absorbance is 0.1, then microcontroller 101 determines that a liquid present is basic, and is present in a low volume. In another example, if a measured absorbance of the 440 nm light is 0.3, then microcontroller determines that the liquid is present in high volume due to a high level of acidity. In this manner, microcontroller 101 need not calculate an intermediate pH level, but rather, can map absorbance or reflectance directly to volume of bodily exudate. Microcontroller 101 can determine expected reflectance, i.e., the amount of light at a particular frequency that is expected to be measured by the photodetector 204, based on an absorbance for that frequency.

The microcontroller 101 can retrieve stored calibration values from memory and determine, from the color and the calibration values, the amount of bodily exudate present in the absorbent article. For example, microcontroller 101 can store a table which maps a given value or range of color to a corresponding amount, or volume of bodily exudate present. Microcontroller 101 can have multiple tables, for example, one for each of a set of different color changing indicators. Additionally, the table can be updated, for example, in the event that a different color changing indicator is to be used.

The wavelength of light source 202 may be altered based on a particular application or color changing indicator. For example, a pH color changing indicator may have a greater response at specific wavelengths, and so the light source 202 may be selected or tuned to emit light at such wavelengths. In this manner, by using light sources with particular wavelengths that are better reflected by the color changing indicator, the system can receive stronger reflected pulsed light signals from the object. This can allow the system to more accurately determine the color of the object and therefore more accurately determine a pH value or a corresponding volume based on the determined color. Such accuracy can be particularly valuable when the color values of the color changing indicator do not change linearly with changes in pH.

Figure 3 depicts an absorbent article with a pH-sensitive color changing indicator and a sensing device, according to certain aspects of the present disclosure. Figure 3 depicts absorbent article system 300, which includes an absorbent article 301, sensor package 310, and color strip 351. In this example, the infant sensing system 100 of Figure 1 is implemented on sensor package 310. Further, in some examples, multiple color detection systems, or multiple light sources and photodetectors for a single multiple color detection system, may be employed at different locations within the absorbent article to better detect the presence of bodily exudate at multiple different locations within the absorbent article.

Color strip 351 is shown as extending down the middle of the absorbent article from one end, shown with straps, to the other. Because bodily exudate can be non-uniformly distributed within an absorbent article, placing the color strip 351 down the middle of the absorbent article increases the chance that the color strip 351 will detect bodily exudate in the absorbent article 301. But color strip 351 can be located in different areas of the absorbent article 301. For example, color strip 351 could be located at the front of the absorbent article, or at an edge of absorbent article 301, or any combination of these or other locations.

As can be seen, sensor package 310 is aligned with color strip 351 such that the light source and photodetector elements are positioned over the color strip 351. In some examples, sensor package 310 can be removable from the absorbent article 301. For example, the sensor package 310 can be adhered to the absorbent article 301 to prevent the sensor package 310 slipping, while allowing its removal.

Absorbent article 301 can be any suitable absorbent article such as a common disposable diaper, a reusable cloth diaper, pantiliner, adult diaper, etc. Color strip 351 is a color changing indicator that is designed to change color in response to contact with a substance having a particular property, such as a pH level. For example, color strip 351 can be Bromocresol green, which changes color based on the pH of a liquid to which the color changing indicator has been exposed. The color of the Bromocresol green strip changes with the pH of bodily exudate detected. Other color changing indicators can be used. The detected pH level can be correlated with a volume of bodily exudate, because the pH level changes as the volume of bodily exudate in the absorbent article changes. Accordingly, a lookup table or function may be used to determine a volume for a given pH level, or color of the color changing indicator.

Sensor package 310 can include the infant sensing system 100 and/or the color sensing system 200, can be included within a flexible, impermeable package. For example, sensor package 310 has a housing that can withstand bodily exudate and feces, and is sufficiently thin as to not cause discomfort to a wearer of the absorbent article. Sensor package 310 may be fabricated with flexible substrate such as a thin plastic, fluoroelastomer, or tpsiv.

Sensor package 310 can be placed in the absorbent article in various different ways. In an aspect, sensor package 310 may be removed and inserted in a new absorbent article. Sensor package 310 can be covered with a material or pouch that is washable or can be wiped. For example, sensor package 310 can be inserted into an absorbent article or adhered to the inside of the absorbent article. Sensor package 310 can also be inserted into a pocket or pouch inside the absorbent article. Such a pocket or pouch can be hermetically sealed, for example, in transparent plastic that allows light to pass through. Sensor package 310 can also be permanently attached into an absorbent article and discarded after a one-time use. Sensor package 310 can also be adhered to the outside of the absorbent article via velcro or similar material.

Figures 4A and 4B depict an example layout of a sensor system that can be placed in or on the outer surface of an absorbent article, according to certain aspects of the present disclosure. Figure 4A represents a top-down view of an example of a sensor layout for sensor package 400. Figure 4B represents a bottom-up view of an example of a sensor layout for sensor package 400. Sensor package 400 can be used in conjunction with the absorbent article 301 depicted in Figure 3, e.g. to perform the functions associated with the color sensing application discussed above. A movement sensor (not shown) may be included in the sensor package 400 or provided separately to provide the functions associated with the activity classification application discussed above.

As depicted, the bottom is the side that is positioned to face and align with the color strip 351. The sensor system shown in Figure 4, when placed in an absorbent article, by detecting a color of a color changing indicator in the absorbent article, can determine a presence and volume of bodily exudate present in the absorbent article in conjunction with an internal system such as microcontroller 101 that can map color to bodily exudate volume.

Sensor package 400 includes a battery 402 and one or more color detector cells 420a-n. Sensor package 400 may also include a switch 404, two electrical connectors 440-441, a volatile organic compound ("VOC") sensor 410, a temperature sensor 411, a humidity sensor 412, an additional ambient light sensor 414, processor 206, microcontroller 101, or transceiver 212. Additional ambient light sensor 414 can be used in conjunction with the photodetectors to improve or augment the light detecting capability of sensor package 400. Some aspects may not include all of the components described above, or include variants thereof.

In addition, the sensor package 400 can cause an alarm, such as an audible beep, based on a threshold level of bodily exudate being detected. Accordingly, sensor package 400 can include a speaker or other audio output device. Sensor package 400 can also cause a transmission of an alert to another device, for example, operated by a caretaker. In another aspect, sensor package 400 can transmit an alert to another device. Sensor package 400 can include a transmitter or transceiver capable of transmitting a radio signal to an external device. Color sensing application 111 operating on microcontroller 101 can also log events, such as when bodily exudate is detected, to memory for later transmission to a caregiver.

Sensor package 400 can include one or more color detector cells 420an. For example, multiple color detector cells 420a-n can increase the ability of the sensor package 400 to detect changes in bodily exudate across the absorbent article. Because bodily exudate may not be distributed uniformly in an absorbent article, the color of color strip 351 may not change uniformly along the length of the color changing indicator. Additionally, the presence of multiple color detector cells 420a-n enables a calculation of multiple data points to more accurately estimate the total load.

Each color detector cell 420a-n includes a light source such as an LED and a photodetector such as a photodiode. In some aspects, as discussed further with respect to Figure 4, a color detector cell may include multiple light sources or multiple photodetectors. Each color detector cell 420a-n detects light reflected by object 251 such as a color strip 351, such as ambient light or pulsed light from the light source(s). The output of each color detector cell 420a-n is provided to a processor 206. The output of processor 206 can be provided to microcontroller 101. In some examples, each color detector cell 420a-n may have a dedicated processor 206, while in some examples, multiple color detector cells 420a-n may be connected to a common processor.

Sensor package 400 can include a switch 404 to activate or deactivate the sensor package 400. The switch 404 can be any suitable switch, such as a rocker-style on/off switch that connects the battery 402 to the electronics in sensor package 400 such as the color detector cells 420a-n and sensors 410-414. Switch 404 can also be a pushbutton switch that activates power from battery 402 to sensor package 400 for a period of time. Sensor package 400 can be configured to automatically turn off to save battery power. In an aspect, in conjunction with microcontroller 101, sensor package can be activated remotely. For example, a user can prompt an external device with a voice command, which causes the external device to transmit a request for a status of the absorbent article to the microcontroller 101 via a wireless connection, or a request to turn on or turn off the sensor package 400.

Sensor package 400 can include one or more electrical connectors such as electrical connectors 440-441. Electrical connectors 440 and 441 can be used to debug the sensor package 400, calibrate the sensor package 400, reset the sensor package 400 to factory settings, upgrade software on the sensor package 400, etc.

As discussed with respect to Figure 1, processor 206 can discern a color of an object such as a color changing indicator. Microcontroller 101 can execute an application such as color sensing application 111 that can perform calibration of the detected color value. Transceiver 212 can notify an external device if the sensor package 400 detects the presence of bodily exudate in an absorbent article.

In an aspect, sensor package 400 can also include a VOC sensor 410. VOC sensor 410 can detect the presence of volatile organic compounds such as feces from a bowl movement or VOCs present in blood. In conjunction with data obtained from color detector cells 420a-n, the VOC sensor 410 can provide additional information to microcontroller 101 based on one or more detected volatile organic compounds.

In an aspect, sensor package 400 can also include a temperature sensor 411. Temperature sensor 411 can detect heat from substances such as bodily exudate. In conjunction with data obtained from color detector cells 420a-n, the temperature sensor 411 can provide additional information such as a temporary increase in temperature to microcontroller 101. Because a notification of a temporary increase in temperature can indicate a presence of bodily exudate, such information can improve the accuracy and reliability of the detection.

In another aspect, sensor package 400 can also include a humidity sensor 412. Humidity sensor 412 can detect the presence of humidity, e.g., from bodily exudate. In conjunction with data obtained from color detector cells 420a-n, humidity sensor 412 can provide additional information such as a notification of a temporary increase in humidity to microcontroller 101. Because a temporary increase in temperature can indicate a presence of bodily exudate, such information can improve the accuracy and reliability of the detection.

In a further aspect, sensor package 400 can also include additional ambient light sensor 414. Additional ambient light sensor 414 can be placed, as shown, oriented away from the color detector cells 420a-n to more accurately detect the ambient light. In conjunction with data obtained from color detector cells 420a-n, additional ambient light sensor 414 can provide additional information to microcontroller 101 that allows microcontroller 101 to better disambiguate the contribution of ambient light to the color of the color changing indicator. Additional ambient light sensor 414 can also provide the microcontroller 101 with information as to whether an infant who is wearing an absorbent article in which the sensor package 400 is placed is in a dark room. For example, sensor package 400 can provide an indication or a notification to a caregiver that the light in a baby's room is either on or off.

As discussed, sensor package 400 can include multiple color detector cells 420a-n. The presence of more than one color detector cell 420a-n allows for increased accuracy and reliability. For example, one color detector cell 420a-n could become obstructed by an object, rendering detected values from that cell unusable, or because bodily exudate may not be evenly distributed in an absorbent article, and therefore not evenly distributed on a color changing indicator, the use of more than one of color detector cell 420a-n increases the probability that one of the color detector cells 420a-n detects bodily exudate. In this manner, additional color detector cells 420a-n help add robustness in the case that any one of color detector cell 420a-n fails or is misaligned. Further, the additional of more sells 420a-n can provide additional local information that may help estimate total load. In contrast, fewer color detector cells 420a-n can simplify the overall system architecture and may also lower power consumption.

In another example, in a system with three detector cells 420a-c, if one detector cell 420a returns a color measurement that is inconsistent with detector cells 420b and 420c, then microcontroller 101 can ignore the measurements from detector cell 420a.

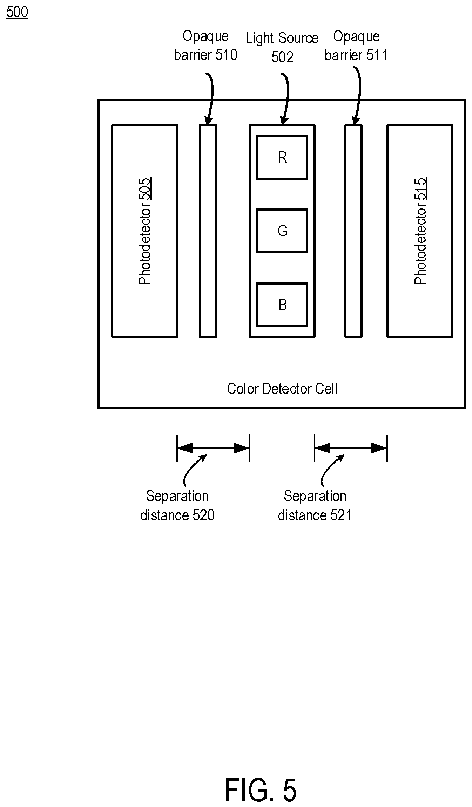

Figure 5 depicts an example color detector cell configuration, according to certain aspects of the current disclosure. As discussed, a sensor system such as sensor package 400 includes one or more color detection cells 420a-n. Figure 5 shows an color detector cell 500 in more detail.

Color detector cell 500 includes two photodetectors, photodetector 505 and photodetector 515, light source 502, opaque barrier 510, and opaque barrier 511. Light source 502 can be any suitable light source according to this disclosure. As shown, light source 502 includes a red, a blue, and a green light source, though different numbers and types of light sources 502 may be used according to different examples, which can allow the light sources can be turned on and off, i.e., pulsed, separately. Pulsing the light sources 502 that emit different colors separately allows color detector cell 500 to tailor the light output to a specific wavelength of light. For example, a particular color changing indicator may be more responsive to a specific wavelength of light at a specific pH level.

Photodetectors 505 and 515 can be any suitable photodetector according to this disclosure. Photodetectors 505 and 515 are connected to the processor 206. A separation distance 520 between the light source 502 and the photodetector 505 and separation distance 521 between light source 502 and photodetector 515 can be adjusted based on the application. In particular, the closer the light source 502 and a photodetector 505 or 515 are together, the greater the portion of light received at the photodetectors from the light source 502 (and less from ambient light 250). As an example only, separation distance 520 and separation distance 521 can be adjusted from 0.1 mm to 2 mm in separation. Other distances and configurations are possible. As a distance increases, all else being equal, the intensity of the light from the light source received at the photodetector decreases. Additionally, as the distance increases, the focal area being measured increases. As the distance decreases, the sensor is more focused on a smaller area directly under the sensor.

As shown, two photodetectors 505 and 515 are used. Photodetectors 505 and 515 can be positioned to be parallel to each other. In this configuration, the combination of photodetectors 505 and 515 provides a stronger output signal to the processor 206 than otherwise. Using more than one photodetector also provides an advantage in that error can be reduced if the sensor system is misaligned with respect to the object, e.g., color strip 351.

Color detector cell 500 can include one or more opaque barriers 510-511 positioned between the light source 502 and the photodetectors 505, 515. The opaque barriers 510-511 reduce the amount of light from light source 502 that travels directly to the photodetector 505 without reflecting off of the object. Opaque barriers 510-511 can be poron or similar material. In an aspect, the photodetectors 505 or 515 can include such an opaque barrier, or an opaque housing of the photodetector 505 or 515 can be extruded in such a manner that the opaque housing is located between the LED and photodiodes. In an aspect, the opaque barriers 510-511 are omitted to simplify the design.

Figure 6 is a flowchart that describes a method of detecting color, according to certain aspects of the present disclosure. The example method of Figure 6 will be described with respect to the color sensing application 111 of Figures 1 or 2; however, any suitable color detection system according to this disclosure may be employed according to different examples. Further, the operations described with respect to Figure 6 can be performed by an external device such as a monitor device connected to infant sensing system 100 via a wireless or an external server.

At block 601 of method 600, color sensing application 111 obtains a first measurement of ambient light received from the photodetector. Photodetector 204 detects the ambient light present and outputs a representation of the color of the light or a representation of an intensity of broad-spectrum light that is present. For example, photodetector 204 can create an electrical output that is proportional to the wavelength or the intensity of the received light. In an aspect, the photodetector 204 can provide three outputs that each correspond to red, green, or blue: a first that is proportional to an amplitude of red in the received light, a second that is proportional to an amplitude of green in the received light, a third that is proportional to an amplitude of blue in the received light.

Photodetector 204 provides the first measurement of light to the processor 206. In this example, the first light measurement is taken while a light source 202 is off and represents ambient light reflected from the object 251. The first light measurement can represent an intensity of broad-spectrum light.

The steps of method 600 can be performed by sensor package 400 of Figure 4 placed in an absorbent article as described with respect to Figure 3. Because sensor package 400 can include one or more color detector cells 420a-n, in an aspect in which more than one color detector cell 420a-n are present, sensor package 400 can measure a level of ambient light at multiple photodetectors. The photodetector in each color detector cell 420a-n can independently perform the steps 601-605.

At block 602 of method 600, the color sensing application 111 causes the light source to transmit of light on an object. More specifically, processor 206 activates light source 202 for a predetermined pulse time interval. In this example, the infant sensing system 100 only includes one light source 202. But in some examples, multiple light sources may be pulsed simultaneously or individually. For example, aspects using sensor package 400 may include more than one color detector cell 420an. The light source in each color detector cell 420a-n may be pulsed separately or together with the other light sources.

At block 603 of method 600, the color sensing application 111 obtains a second measurement from the photodetector during the transmission, the second measurement including the ambient light and the transmitted light reflected from the object. Processor 206 obtains a second measurement of light during the time interval that the pulse from light source 202 is on. The second measurement includes the ambient light and the light from the pulsed light source 202. In an aspect such as sensor package 400, the photodetector in each color detector cell 420a-n each obtains a second measurement of light. Color sensing application 111 uses the first and second measurements to determine the color of an object.

In an aspect, color sensing application 111 can obtain more than one measurement with the ambient light and the pulsed light present. Processor 206 can average the multiple measurements together to form one single measurement that can be used as a second measurement.

At block 604 of method 600, color sensing application 111 determines a normalized measurement of the reflected light by removing an ambient light signal from the second measurement based on the first measurement. Removal can be performed in the analog domain or the digital domain.

For example, processor 206 can remove the first measurement of light from the second measurement of light by filtering in the analog domain. For example, the processor 206 subtracts the first measurement, representing the ambient light, from the second measurement, representing the ambient light combined with the reflected light from light source 202. The result of the subtraction is the light reflected from the object 251, such as a color changing indicator.

Processor 206 can operate in the digital domain. For example, processor 206 converts the first measurement into a digital or numeric representation of the red, green, and blue levels. Processor 206 converts the second measurement into a digital or numeric representation of the red, green, and blue levels. Processor 206 computes a new red level by subtracting the first measurement from the red level of the second measurement, a new green level by subtracting the first measurement from the green level of the second measurement, and a new blue level by subtracting the first measurement from the blue level of the second measurement. The new red, green, and blue levels represent the color of the light reflected from the object.

At block 605 of method 600, the color sensing application 111 determines, based on the normalized measurement, one of (i) a presence of bodily exudate or (ii) a volume of bodily exudate present. Processor 206 outputs the color of the object and provides the color to microcontroller 101. The color sensing application 111, executing on microcontroller 101, receives the color value from processor 206 and uses a data structure such as a table to determine a presence of bodily or a value representing a volume of bodily exudate. Microcontroller 101 may store several tables, for example, one table which facilitates the mapping of a color on a color changing indicator such as Bromocresol green, to a pH level, and another table that facilitates the mapping of a color changing indicator to a measure or presence of a volume of bodily exudate.

Additionally, as discussed, color sensing application 111 can perform color calibration. Color sensing application 111 can convert the red, green, and blue levels to hue, saturation, and lightness/value and perform calculations on the hue, saturation, and lightness/value. Color calibration can be implemented via a table. For example, for a given triple of red, green, and blue, adjust the values by certain amount. Color calibration can also be performed in a different domain such as hue, saturation, and lightness, or hue, saturation, and value.

In an aspect, color sensing application 111 can determine the presence of bodily exudate in the presence of movement. For example, sensor package 400 caused to be moved by an infant at the same time as color sensing application 111 is performing measurements. In this case, color sensing application can use a known responsiveness of the absorbent article or color strip at two or more different wavelengths of light to determine a presence of exudate. In an example color sensing application 111 can detect that a response to red light is greater than a response to blue light even in the presence of motion.

In a further aspect, color sensing application 111 can detect when an absorbent article is not attached to an infant. In this case, the sensor responsiveness changes below a threshold, which is detected by color sensing application 111.

Figure 7 is a flowchart of an exemplary method used to determine activity from a movement sensor, according to certain aspects of the present disclosure. Method 700 can be implemented by activity classification application 116. Further, the operations described with respect to Figure 7 can be performed by an external device such as a monitor device connected to infant sensing system 100 via a wireless or an external server.

At block 701 of method 700, activity classification application 116 receives, from movement sensor 130, a time series of data including an inertial measurement for each of a set of time periods. Inertial measurements can include acceleration or angular velocity. For example, an accelerometer can provide a triplet of numerical values corresponding to the x, y, and z directions. Activity classification application 116 periodically samples the accelerometer to create a time series of data. Processor 206 annotates each triplet with a timestamp, creating a pair that includes sensor measurement and timestamp. Activity classification application 116 can also sample the gyroscope on a periodic basis. In conjunction with the measurement data from the accelerometer, activity classification application 116 can determine a set of data that includes a gyroscope measurement, e.g. angular velocity, an accelerometer measurement, e.g., a triplet of x-y-z values, and a timestamp.

In an aspect, activity classification application 116 analyzes measurement data in real-time and can update an activity measurement function or the predictive model in real-time. Alternatively, activity classification application 116 can analyze a block of samples at a time. For example, activity classification application 116 can buffer the pairs until a threshold number of pairs have been received and then analyze movement over a window of time.

At block 702 of method 700, activity classification application 116 calculates, from a subset of the time series of data, an activity function from statistical data derived from the inertial measurement. Statistical data can include data such as (i) a statistical variance of the inertial measurement or (ii) a root-mean-square of the inertial measurement. Activity classification application 116 uses an activity measurement function in order to determine activity level. Different measurements of activity can be derived. For example, activity classification application 116 can calculate the statistical variance, standard deviation, or the root mean square (RMS) of the signal. Activity classification application 116 can use another customized metrics based on the accelerometer or gyroscope data. For example, a customized metric that quantifies the level of activity *A* can be calculated for a given number *n* of samples with the following function, where *Sx, Sy,* and *Sz* are the sum of the square differences from the respective means in the *x, y,* and z dimensions respectively:

$$A = \sqrt{\frac{(Sx)^2 + (Sy)^2 + (Sz)^2}{n}}$$

At block 703 of method 700, activity classification application 116 determines an activity indicated by the subset of time series data based on a measure from the activity function being greater than a first threshold and less than a second threshold. Activity classification application 116 can determine an activity such as sleeping or awake based on a level of activity being with a range of values. For example, if the activity function measures a level of activity below a first threshold but above zero, then activity classification application 116 determines that the infant is in light sleep. If the activity function measures a level of movement below a second, lower, threshold, then the monitor application determines that the infant is in a deep sleep. Activity classification application 116 can use a state machine to determine activity states.

As discussed, in an aspect, activity classification application 116 uses a predictive model to determine the infant's activity in addition to or instead of algorithms or state machines. Activity classification application 116 provides the accelerometer measurements, the gyroscope measurements, or the output of an activity measurement function to the predictive model. In embodiments of the invention, the predictive models are machine learning models such as decision tree classifiers or regression models. A predictive model is trained to determine whether a wearer of the sensor is feeding on the left hand side, feeding on the right hand side, sleeping, awake and playing on its back, being held, or sitting. Other detectable activities may include sitting, playing, crawling, walking, etc. Activity classification application 116 can provide data for one or more periods of time to the predictive model. In this manner, predictive model may determine an activity based on present or past activity level.

Figure 8 is a flowchart of an exemplary method used to determine activity from a movement sensor by using a predictive model, according to certain aspects of the present disclosure. Further, the operations described with respect to Figure 8 can be performed by an external device such as a monitor device connected to infant sensing system 100 via a wireless or an external server.

At block 801 of method 800, activity classification application 116 receives, from a movement sensor, a time series of data including an inertial measurement for each of a set of time periods. At block 801, activity classification application 116 receives the time series of data generally as described with respect to block 701.

At block 802 of method 800, activity classification application 116 calculates, from the time series data, an activity function such as (i) a statistical variance of the of the inertial measurement or (ii) a root-mean-square of the inertial measurement. At block 802, monitor application uses an activity measurement function generally as described with respect to block 702.

At block 803 of method 800, activity classification application 116 provides the activity function the (i) statistical variance or (ii) the root mean square of the inertial measurement to a predictive model trained to identify an activity a list of activities. More specifically, activity classification application 116 provides sensor measurements or the output of the activity function to the predictive model.

The predictive model is trained to determine activity from measurements that indicate movement. The predictive model can be trained by providing training data to the predictive model and iteratively adjusting internal parameters of the model to reduce error. Training data can include pairs of movement data and a ground truth (an identified activity). For example, training data can include a set of movements and a training data tag "sleeping" or "feeding." Then, the predictive model learns to associate particular movement patterns with an activity.

The trained predictive model determines, based on its training, from a predefined set of classes, to which class the activity belongs. An exemplary list of activity classes includes feeding on the left side, feeding on the right side, sleeping, awake but playing on back, being held, and sitting. Other training classes are possible. For example, the predictive model can be trained to distinguish deep sleep from light sleep, and activities such as crawling, rolling, sitting up, feeding, or nursing from each other. For example, activity classification application 116 may include a predictive model that is trained to distinguish between asleep, awake, stirring, or settled states, and another that is trained to distinguish between light sleep and deep sleep. Stirring represents a state in which an infant is moving more than a first threshold amount and settled represents a state in which the infant has calmed down and is moving less than a second threshold amount.

At block 804 of method 800, activity classification application 116 receives, from the predictive model, a determination of an activity corresponding to the subset of time series data. For example, the predictive model provides a prediction to activity classification application 116 from one of the trained categories such as feeding on the left hand side, feeding on the right hand side, sleeping, awake and playing on its back, being held, or sitting.

Figure 9 is a flowchart that describes a method 900 of detecting a volume of bodily exudate in an absorbent article, according to certain aspects of the present disclosure. Method 900 is explained from the perspective of diaper loading application 102, activity classification application 116, and color sensing application 111, but as can be appreciated, different steps of method 900 can be performed by these or other applications. Additionally, one application can perform all of the steps. Further, the operations described with respect to Figure 9 can be performed by an external device such as a monitor device connected to infant sensing system 100 via a wireless or an external server.

At block 901 of method 900, color sensing application 111 obtains a first measurement of ambient light received from a photodetector while a light source is off. At block 901, color sensing application 111 performs similar functions as described with respect to block 601 of method 600.

At block 902 of method 900, color sensing application 111 obtains a second measurement from the photodetector while the light source is transmitting light. The second measurement includes a measurement of the ambient light and the transmitted light reflected from an absorbent article. At block 902, color sensing application 111 performs similar functions as described with respect to blocks 601-602 of method 600.

At block 903 of method 900, diaper loading application 115 determines a normalized measurement of the light reflected from an absorbent article by removing an ambient light signal from the second measurement based on the first measurement. At block 903, diaper loading application 115 causes color sensing application 111 to perform similar functions as described with respect to block 604 of method 600.

At block 904 of method 900, diaper loading application 115 determines, from the normalized measurement, a presence of urine in the absorbent article. At block 904, diaper loading application 115 causes color sensing application 111 to perform similar functions as described with respect to block 605 of method 600.

At block 905 of method 900, diaper loading application 115 determines a degree of fullness of the absorbent article. The degree of fullness reflects an amount of storage space in an absorbent article that is filled or has absorbed bodily exudate relative to a total amount of storage space that can be filled with bodily exudate. In some cases, the degree of fullness of the absorbent article is derived from a volume of urine present in the diaper. The volume can be determined using different inputs such as (i) an elapsed time since the presence of urine, (ii) when the diaper was changed, and (iii) a state of the infant (e.g., awake or sleeping).

A diaper replacement can be indicated by a caregiver, e.g., via a user interface or other input to the infant sensing system. Alternatively, diaper loading application 115 can detect a presence of a new diaper by detecting a removal of the sensor from the infant's diaper, or a decrease in or absence of wetness measured.

Understanding the amount of time in asleep and/or awake states facilitates improved predictions. For example, infants may urinate at a slower frequency and quantity during the night relative to the day. Additionally, with more accurate predictions in this respect, the infant sensing system has an added benefit of allowing a caregiver to sleep longer if a diaper change is not imminently necessary. As explained with respect to Figures 7-8, a movement sensor in conjunction with a predictive model is used by activity classification application 116 to determine whether an infant is asleep, awake, resting, etc. hence, at block 905, example operations include operations performed in methods 700 and/or 800.

Additionally, in some cases, the diaper loading application 115 receives an input about the particular type, brand, or size (e.g., standard sizes such as 1, 2, 3. etc.) of diaper being used or whether the diaper is a regular (daytime) diaper or an overnight diaper. Overnight diapers may have a greater absorption capacity. Further, diaper loading application 115 can receive demographic information about the infant such as age, gender, weight, etc., which can be used for the basis of predictions. For example, larger infants may urinate more, resulting in a diaper needing to be replaced sooner than with a smaller infant.

Diaper loading application 115 can also determine a time until the absorbent article is full. For example, diaper loading application 115 access a capacity of the diaper (e.g., a volume of liquid that can theoretically be stored in the diaper), calculate a rate of volume increase (e.g., based on frequency and amount if urine events since the diaper was replaced) and calculate a time at which the diaper will be full. Diaper loading application 115 can cause infant sensing system 100 to send an alert on or before the time to remind a caregiver to tend to the infant.

Diaper loading application 115 can model how much liquid a diaper is expected to hold based on a particular size of the diaper, type of diaper, or other parameters. Then, based on the model, the diaper loading application can evaluate a loading of a particular diaper over time and based on one or more events. For example, the application can consider (1) when the diaper was replaced with a new one (e.g., how long the current diaper has been on an infant), (2) whether the diaper is wet (or when it first turned wet), (3) the amount of time spent in asleep or awake states since the diaper became wet, or (4) other data such as diaper type.

In aspects of the invention, statistical methods are used. For example, the diaper loading application 115 solves the function with one or more regression models (e.g., linear, quadratic, etc.) or machine learning models (e.g., a decision tree classifier or other classification model). By solving the model, diaper loading application 115 determines a volume of urine present in the diaper.

Figure 10 is a diagram depicting an example computing system for performing functions related to color detection and detection of bodily exudate, according to some aspects of the present disclosure. Some or all of the components of the computing system 1000 can belong to microcontroller 101 or the processor 206 of Figure 1. For example, the color sensing application 111 may operate on the computing system 1000. The computing system 1000 includes one or more processors 1002 communicatively coupled to one or more memory devices 1014. The processor 1002 executes computer-executable program code, which can be in the form of non-transitory computer-executable instructions, stored in the memory device 1014, accesses information stored in the memory device 1014, or both. Examples of the processor 1002 include a microprocessor, an application-specific integrated circuit ("ASIC"), a field-programmable gate array ("FPGA"), or any other suitable processing device. The processor 1002 can include any number of processing devices, including one.

The memory device 1014 includes any suitable computer-readable medium such as electronic, optical, magnetic, or other storage device capable of providing a processor with computer-readable instructions or other program code. Non-limiting examples of a computer-readable medium include a magnetic disk, a memory chip, a ROM, a RAM, an ASIC, optical storage, magnetic tape or other magnetic storage, or any other medium from which a processing device can read instructions. The instructions may include processor-specific instructions generated by a compiler or an interpreter from code written in any suitable computer-programming language, including, for example, C, C++, C#, Visual Basic, Java, Python, Perl, JavaScript, and ActionScript.

The computing system 1000 may also include a number of external or internal devices such as input or output devices. For example, the computing system 1000 is shown with an input/output ("I/O") interface 1010 that can receive input from input devices or provide output to output devices. A bus 1006 can also be included in the computing system 1000. The bus 1006 can communicatively couple one or more components of the computing system 1000 and allow for communication between such components.

The computing system 1000 executes program code that configures the processor 1002 to perform one or more of the operations described above with respect to Figures 1-5. The program code of the color sensing application 111, diaper loading application 115, or activity classification application 116, which can be in the form of non-transitory computer-executable instructions, can be resident in the memory device 1014 or any suitable computer-readable medium and can be executed by the processor 1002 or any other one or more suitable processor. Execution of such program code configures or causes the processor(s) to perform the operations described herein with respect to the microcontroller 101. In additional or alternative aspects, the program code described above can be stored in one or more memory devices accessible by the computing system 1000 from a remote storage device via a data network. The microcontroller 101 and any processes can use the memory device 1014. The memory device 1014 can store, for example, additional programs, or data used by the applications executing on the processor 1002 such as the color sensing application 111.

The computing system 1000 can also include at least one network interface 1004. The network interface 1004 includes any device or group of devices suitable for establishing a wired or wireless data connection to one or more data networks. Non-limiting examples of the network interface 1004 include an Ethernet network adapter, WiFi network, Bluetooth, or Bluetooth Low Energy (BLE), a modem, or the like. The computing system 1000 is able to communicate with one or more other computing devices or computer-readable data sources via a data network using the network interface 1004.

Numerous specific details are set forth herein to provide a thorough understanding of the claimed subject matter. However, those skilled in the art will understand that the claimed subject matter may be practiced without these specific details. In other instances, methods, apparatuses, or systems that would be known by one of ordinary skill have not been described in detail so as not to obscure claimed subject matter.

Unless specifically stated otherwise, it is appreciated that throughout this specification discussions utilizing terms such as "processing," "computing," "calculating," "determining," and "identifying" or the like refer to actions or processes of a computing device, such as one or more computers or a similar electronic computing device or devices, that manipulate or transform data represented as physical electronic or magnetic quantities within memories, registers, or other information storage devices, transmission devices, or display devices of the computing platform.

The system or systems discussed herein are not limited to any particular hardware architecture or configuration. A computing device can include any suitable arrangement of components that provide a result conditioned on one or more inputs. Suitable computing devices include multi-purpose microprocessor-based computer systems accessing stored software that programs or configures the computing system from a general purpose computing apparatus to a specialized computing apparatus implementing one or more aspects of the present subject matter. Any suitable programming, scripting, or other type of language or combinations of languages may be used to implement the teachings contained herein in software to be used in programming or configuring a computing device.

## Claims

1. A computer-implemented method for determining a degree of fullness of a wearable absorbent article, the method comprising:

    obtaining a first measurement of ambient light received from a photodetector while a light source is off;
    obtaining a second measurement from the photodetector while the light source is transmitting light, the second measurement comprising a measurement of the ambient light and the transmitted light reflected from an absorbent article;
    determining a normalized measurement of light reflected from an absorbent article by removing an ambient light signal from the second measurement based on the first measurement of ambient light;
    determining, from the normalized measurement of light, a presence of bodily exudate in the absorbent article;
    determining, from a time series of data including an inertial measurement for each of a set of time periods, an activity state, wherein determining the activity state comprises calculating statistical data derived from the inertial measurements, providing the inertial measurements and statistical data to a predictive model, and receiving from the predictive model a determined activity state; and
    determining a degree of fullness of the absorbent article, wherein determining the degree of fullness comprises using one or more of a regression model or a machine learning model to solve a function that has inputs indicative of (i) an elapsed time since the determination of the presence of bodily exudate determined from the normalized measurement of light and (ii) the activity state.

2. The computer-implemented method of claim 1, further comprising:

    calculating a time until the absorbent article is expected to be full by using a regression model to solve a function that has inputs indicative of the elapsed time and the activity state; and
    outputting, on or before the calculated time, a notification recommending replacement of the absorbent article.

3. The computer-implemented method of claim 1 or 2, wherein determining the degree of fullness is based on a size of the absorbent article.

4. The computer-implemented method of any one of claims 1 to 3, wherein determining the degree of fullness is further based on (iii) an additional elapsed time since the absorbent article was placed on an infant.

5. The computer-implemented method of any one of claims 1 to 4, wherein the activity state comprises one of (i) asleep or (ii) awake.

6. The computer-implemented method of any one of claims 1 to 5, wherein the absorbent article is a diaper worn by an infant, and wherein the method further comprises accessing a set of demographics about the infant, and wherein determining the degree of fullness is based in part on the demographics.

7. The computer-implemented method of any one of claims 1 to 6, further comprising accessing a type of the absorbent article, wherein the type is one of (i) a nighttime diaper or (ii) a daytime diaper, and wherein determining the degree of fullness is based in part on the type.

8. The computer-implemented method of any one of claims 1 to 7, wherein the second measurement comprises separate measurements of red light, green light, and blue light, wherein determining the normalized measurement of light comprises determining a separate intensity of red light, green light, and blue light, and wherein determining the degree of fullness is based on the intensities of the red, green, and blue lights.

9. The computer-implemented method of any one of claims 1 to 8, including selecting a wavelength for the light transmitted by the light source based on a responsiveness of the absorbent article to different wavelengths of light.

10. The computer-implemented method of any one of claims 1 to 9, wherein the absorbent article comprises a printed or coated region comprising a color changing indicator.

11. The computer-implemented method of any one of claims 1 to 10, further comprising:

retrieving, from a memory, a stored color calibration value; and
determining, based on the normalized measurement of light and the stored color calibration value, a color of the absorbent article, wherein the stored color calibration value is determined using a white colored object and the determining the color comprises white level correction.

12. A system for determining a volume of bodily exudate in an absorbent article, the system comprising:

a light source;
a photodetector;
a movement sensor; and
a processor that is configured to:

obtain a first measurement of ambient light received from the photodetector while the light source is off;
obtain a second measurement from the photodetector while the light source is transmitting light, the second measurement comprising a measurement of the ambient light and the transmitted light reflected from an absorbent article;
determine a normalized measurement of light reflected from an absorbent article by removing an ambient light signal from the second measurement based on the first measurement of ambient light;
obtain, from the movement sensor, a time series of data including an inertial measurement for each of a set of time periods;
transmit, to an external device also comprised in the system, (i) the normalized measurement of light and (ii) the time series of data, wherein the external device upon receiving (i) the normalized measurement of light and (ii) the time series of data, is configured to:

determine an activity state by calculating statistical data derived from the inertial measurements, providing the inertial measurements and statistical data to a predictive model, and receiving from the predictive model a determined activity state; and
determine the degree of fullness using one or more of (i) a regression model or (ii) a machine learning model to solve a function that has inputs indicative of (i) an elapsed time since the determination of the presence of bodily exudate determined from the normalized measurement of light and (ii) the activity state; and
receive, from the external device, a degree of fullness of the absorbent article.

13. The system of claim 12, wherein the external device is further configured to calculate a time until the absorbent article is expected to be full by using a regression model to solve a function that has inputs indicative of the elapsed time and the activity state.

14. The system of claim 12 or 13, wherein the external device is further configured to determine the degree of fullness based on (iii) an additional elapsed time since the absorbent article was placed on an infant.

15. A system comprising:

a computer-readable medium storing non-transitory computer-executable instructions; and
a processing device communicatively coupled to the computer-readable medium for executing the non-transitory computer-executable instructions, wherein executing the non-transitory computer-executable instructions configures the processing device to perform the computer-implemented method of any one of claims 1 to 11.

**Patentansprüche**

1. Computerimplementiertes Verfahren zur Bestimmung des Vollheitsgrads eines tragbaren absorbierenden Artikels, wobei das Verfahren Folgendes umfasst:

   Erhalten einer ersten Messung von Umgebungslicht, die von einem Photodetektor erhalten wird, während eine Lichtquelle ausgeschaltet ist;
   Erhalten einer zweiten Messung vom Photodetektor, während die Lichtquelle Licht aussendet, wobei die zweite Messung eine Messung des Umgebungslichts und des ausgesendeten Lichts, das von einem absorbierenden Artikel reflektiert wird, umfasst;
   Bestimmen einer normalisierten Messung von Licht, das von einem absorbierenden Artikel reflektiert wird, durch Entfernen eines Umgebungslichtsignals von der zweiten Messung basierend auf der ersten Messung von Umgebungslicht;
   Bestimmen, aus der normalisierten Messung von Licht, des Vorhandenseins eines Körperexsudats im absorbierenden Artikel;
   Bestimmen, aus einer Zeitreihe von Daten, die eine Inertialmessung für jeden aus einem Satz von Zeiträumen umfasst, eines Aktivitätszustands, wobei das Bestimmen des Aktivitätszustands das Berechnen von statistischen Daten umfasst, die von den Inertialmessungen abgeleitet sind, Bereitstellen der Inertialmessungen und der statistischen Daten an ein Vorhersagemodell und Empfangen eines bestimmten Aktivitätszustands vom Vorhersagemodell; und
   Bestimmen eines Vollheitsgrads des absorbierenden Artikels, wobei das Bestimmen des Vollheitsgrads das Verwenden eines oder mehrerer aus einem Regressionsmodell oder einem Modell für maschinelles Lernen zum Lösen einer Funktion umfasst, die Eingaben aufweist, die (i) eine seit der Bestimmung des Vorhandenseins von Körperexsudat, das durch die normalisierte Messung von Licht bestimmt wurde, vergangene Zeit und (ii) den Aktivitätszustand umfassen.

2. Computerimplementiertes Verfahren nach Anspruch 1, das weiters Folgendes umfasst:

   Berechnen einer Zeit, bis zu der erwartet wird, dass das absorbierende Material voll ist, unter Verwendung eines Regressionsmodells zum Lösen einer Funktion, die Eingaben aufweist, die die vergangene Zeit und den Aktivitätszustand angeben; und
   Ausgeben einer Benachrichtigung, die den Austausch des absorbierenden Gegenstands empfiehlt, zur oder vor der berechneten Zeit.

3. Computerimplementiertes Verfahren nach Anspruch 1 oder 2, wobei das Bestimmen des Vollheitsgrads auf der Größe des absorbierenden Artikels basiert.

4. Computerimplementiertes Verfahren nach einem der Ansprüche 1 bis 3, wobei das Bestimmen des Vollheitsgrads weiters auf (iii) einer weiteren vergangenen Zeit, seit der absorbierende Artikel auf einen Säugling gegeben wurde, umfasst.

5. Computerimplementiertes Verfahren nach einem der Ansprüche 1 bis 4, wobei der Aktivitätszustand eines aus (i) schlafend oder (ii) wach umfasst.

6. Computerimplementiertes Verfahren nach einem der Ansprüche 1 bis 5, wobei der absorbierende Artikel eine Windel ist, die von einem Säugling getragen wird, und wobei das Verfahren weiters das Zugreifen auf einen Satz von demographischen Angaben über den Säugling umfasst und wobei das Bestimmen des Vollheitsgrads teilweise auf den demographischen Angaben basiert.

7. Computerimplementiertes Verfahren nach einem der Ansprüche 1 bis 6, das weiters das Zugreifen auf den Typ des absorbierenden Artikels umfasst, wobei der Typ einer aus (i) einer Nachtwindel oder (ii) einer Tageswindel ist und wobei das Bestimmen des Vollheitsgrads teilweise auf dem Typ basiert.

8. Computerimplementiertes Verfahren nach einem der Ansprüche 1 bis 7, wobei die zweite Messung separate Messungen von rotem Licht, grünem Licht und blauem Licht umfasst, wobei das Bestimmen der normalisierten Messung von Licht das Bestimmen einer separaten Intensität von rotem Licht, grünem Licht und blauem Licht umfasst und wobei das Bestimmen des Vollheitsgrads auf der Intensität des roten, grünen und blauen Lichts basiert.

9. Computerimplementiertes Verfahren nach einem der Ansprüche 1 bis 8, das eine zweite Wellenlänge für Licht, das von der Lichtquelle ausgestrahlt wird, basierend auf der Ansprechempfindlichkeit des absorbierenden Artikels auf unterschiedliche Wellenlängen von Licht umfasst.

10. Computerimplementiertes Verfahren nach einem der Ansprüche 1 bis 9, wobei der absorbierende Artikel einen gedruckten oder beschichteten Bereich umfasst, der einen Farbänderungsindikator umfasst.

11. Computerimplementiertes Verfahren nach einem der Ansprüche 1 bis 10, das weiters Folgendes umfasst:

Abrufen eines gespeicherten Farbkalibrierungswerts aus einem Speicher; und Bestimmen der Farbe des absorbierenden Artikels basierend auf der normalisierten Messung von Licht und dem gespeicherten Farbkalibrierungswert, wobei der gespeicherte Farbkalibrierungswert unter Verwendung eines weißfarbenen Objekts bestimmt wird und das Bestimmen der Farbe eine Weißwertkorrektur umfasst.

12. System zum Bestimmen des Volumens von Körperexsudat in einem absorbierenden Artikel, wobei das System Folgendes umfasst:

eine Lichtquelle;
einen Photodetektor;
einen Bewegungssensor; und
einen Prozessor, der ausgelegt ist, um:

eine erste Messung von Umgebungslicht zu erhalten, die von einem Photodetektor erhalten wird, während eine Lichtquelle ausgeschaltet ist;
eine zweite Messung vom Photodetektor zu erhalten, während die Lichtquelle Licht aussendet, wobei die zweite Messung eine Messung des Umgebungslichts und des ausgesendeten Lichts, das von einem absorbierenden Artikel reflektiert wird, umfasst;
eine normalisierte Messung von Licht, das von einem absorbierenden Artikel reflektiert wird, durch Entfernen eines Umgebungslichtsignals von der zweiten Messung basierend auf der ersten Messung von Umgebungslicht zu bestimmen;
vom Bewegungssensor eine Zeitreihe von Daten zu erhalten, die eine Inertialmessung für jeden aus einem Satz von Zeiträumen umfasst;
(i) die normalisierte Messung von Licht und (ii) die Zeitreihe von Daten zu einer externen Vorrichtung zu übertragen, die ebenfalls im System umfasst ist, wobei die externe Vorrichtung ausgelegt ist, um beim Empfang (i) der normalisierten Messung von Licht und (ii) der Zeitreihen von Daten:

einen Aktivitätszustand durch Berechnen von statistischen Daten zu bestimmen, die von den Inertialmessungen abgeleitet sind, die Inertialmessungen und die statistischen Daten an ein Vorhersagemodell bereitzustellen und einen bestimmten Aktivitätszustand vom Vorhersagemodell zu empfangen; und

den Vollheitsgrad unter Verwendung von einem oder mehreren aus (i) einem Regressionsmodell oder (ii) einem Modell für maschinelles Lernen zum Lösen einer Funktion zu bestimmen, die Eingaben aufweist, die (i) eine seit der Bestimmung des Vorhandenseins von Körperexsudat, die durch die normalisierte Messung von Licht bestimmt wurde, vergangene Zeit und (ii) den Aktivitätszustand umfassen; und
einen Vollheitsgrad des absorbierenden Artikels von der externen Vorrichtung zu empfangen.

13. System nach Anspruch 12, wobei die externe Vorrichtung weiters ausgelegt ist, um eine Zeit, bis zu der erwartet wird, dass das absorbierende Material voll ist, unter Verwendung eines Regressionsmodells zum Lösen einer Funktion, die Eingaben aufweist, die die vergangene Zeit und den Aktivitätszustand angeben, zu berechnen

14. System nach Anspruch 12 oder 13, wobei die externe Vorrichtung weiters ausgelegt ist, um den Vollheitsgrad basierend auf (iii) einer weiteren vergangenen Zeit, seit der absorbierende Artikel auf einen Säugling gegeben wurde zu bestimmen.

15. System, das Folgendes umfasst:

ein computerlesbares Medium, auf dem nichttransitorische, durch einen Computer ausführbare Anweisungen gespeichert sind; und
eine Verarbeitungsvorrichtung, die kommunikativ mit dem computerlesbaren Medium gekoppelt ist, um die nichttransitorischen, durch einen Computer ausführbaren Anweisungen auszuführen, wobei das Ausführen der nichttransitorischen, durch einen Computer ausführbaren Anweisungen die Verarbeitungsvorrichtung konfiguriert, um ein computerimplementiertes Verfahren nach einem der Ansprüche 1 bis 11 auszuführen.

**Revendications**

1. Procédé mis en œuvre par ordinateur pour déterminer un degré de remplissage d'un article absorbant portable, le procédé comprenant les étapes consistant à :

obtenir une première mesure d'une lumière ambiante reçue à partir d'un photodétecteur alors qu'une source de lumière est éteinte ;
obtenir une seconde mesure à partir du photodétecteur pendant que la source de lumière transmet de la lumière, la seconde mesure comprenant une mesure de la lumière ambiante et de la lumière transmise réfléchie par un article absorbant ;

déterminer une mesure normalisée de lumière réfléchie par un article absorbant en supprimant un signal de lumière ambiante de la seconde mesure sur la base de la première mesure de lumière ambiante ;

déterminer, à partir de la mesure normalisée de lumière, une présence d'exsudat corporel dans l'article absorbant ;

déterminer, à partir d'une série temporelle de données incluant une mesure inertielle pour chacune d'un ensemble de périodes de temps, un état d'activité, dans lequel la détermination de l'état d'activité comprend un calcul de données statistiques dérivées des mesures inertielles, une fourniture des mesures inertielles et des données statistiques à un modèle prédictif, et une réception à partir du modèle prédictif d'un état d'activité déterminé ; et

déterminer un degré de remplissage de l'article absorbant, dans lequel la détermination du degré de remplissage comprend une utilisation d'un ou plusieurs parmi un modèle de régression ou un modèle d'apprentissage automatique pour résoudre une fonction qui présente des entrées indicatives (i) d'un temps écoulé depuis la détermination de la présence d'exsudat corporel déterminée à partir de la mesure normalisée de lumière et (ii) de l'état d'activité.

2. Procédé mis en œuvre par ordinateur selon la revendication 1, comprenant en outre les étapes consistant à :

calculer un temps jusqu'à ce que l'article absorbant soit censé être rempli en utilisant un modèle de régression pour résoudre une fonction qui présente des entrées indicatives du temps écoulé et de l'état d'activité ; et

délivrer en sortie, au plus tard au temps calculé, une notification recommandant le remplacement de l'article absorbant.

3. Procédé mis en œuvre par ordinateur selon la revendication 1 ou 2, dans lequel la détermination du degré de remplissage est basée sur une taille de l'article absorbant.

4. Procédé mis en œuvre par ordinateur selon l'une quelconque des revendications 1 à 3, dans lequel la détermination du degré de remplissage est en outre basée sur (iii) un temps écoulé supplémentaire depuis que l'article absorbant a été placé sur un nourrisson.

5. Procédé mis en œuvre par ordinateur selon l'une quelconque des revendications 1 à 4, dans lequel l'état d'activité comprend un parmi (i) endormi ou (ii) éveillé.

6. Procédé mis en œuvre par ordinateur selon l'une quelconque des revendications 1 à 5, dans lequel l'article absorbant est une couche portée par un nourrisson, et dans lequel le procédé comprend en outre un accès à un ensemble de données démographiques concernant le nourrisson, et dans lequel la détermination du degré de remplissage est basée en partie sur les données démographiques.

7. Procédé mis en œuvre par ordinateur selon l'une quelconque des revendications 1 à 6, comprenant en outre un accès à un type de l'article absorbant, dans lequel le type est un parmi (i) une couche de nuit ou (ii) une couche de jour, et dans lequel la détermination du degré de remplissage est basée en partie sur le type.

8. Procédé mis en œuvre par ordinateur selon l'une quelconque des revendications 1 à 7, dans lequel la seconde mesure comprend des mesures séparées de lumière rouge, de lumière verte et de lumière bleue, dans lequel la détermination de la mesure normalisée de lumière comprend une détermination d'une intensité séparée de lumière rouge, de lumière verte et de lumière bleue, et dans lequel la détermination du degré de remplissage est basée sur les intensités des lumières rouge, verte et bleue.

9. Procédé mis en œuvre par ordinateur selon l'une quelconque des revendications 1 à 8, incluant une sélection d'une longueur d'onde pour la lumière transmise par la source de lumière sur la base d'une réactivité de l'article absorbant à différentes longueurs d'onde de lumière.

10. Procédé mis en œuvre par ordinateur selon l'une quelconque des revendications 1 à 9, dans lequel l'article absorbant comprend une région imprimée ou revêtue comprenant un indicateur de changement de couleur.

11. Procédé mis en œuvre par ordinateur selon l'une quelconque des revendications 1 à 10, comprenant en outre les étapes consistant à :

récupérer, à partir d'une mémoire, une valeur d'étalonnage de couleur stockée ; et

déterminer, sur la base de la mesure normalisée de lumière et de la valeur d'étalonnage de couleur stockée, une couleur de l'article absorbant, dans lequel la valeur d'étalonnage de couleur stockée est déterminée à l'aide d'un objet de couleur blanche, et la détermination de la couleur comprend une correction de niveau de blanc.

12. Système pour déterminer un volume d'exsudat corporel dans un article absorbant, le système comprenant :

une source de lumière ;
un photodétecteur ;
un capteur de mouvement ; et
un processeur qui est configuré pour :

obtenir une première mesure de lumière ambiante reçue du photodétecteur lorsque la source de lumière est éteinte ;
obtenir une seconde mesure à partir du photodétecteur pendant que la source de lumière transmet de la lumière, la seconde mesure comprenant une mesure de la lumière ambiante et de la lumière transmise réfléchie par un article absorbant ;
déterminer une mesure normalisée de lumière réfléchie par un article absorbant en supprimant un signal de lumière ambiante de la seconde mesure sur la base de la première mesure de lumière ambiante ;
obtenir, à partir du capteur de mouvement, une série temporelle de données comprenant une mesure inertielle pour chacune d'un ensemble de périodes de temps ;
transmettre, à un dispositif externe également compris dans le système, (i) la mesure normalisée de lumière et (ii) la série temporelle de données, dans lequel le dispositif externe, lors de la réception (i) de la mesure normalisée de lumière et (ii) de la série temporelle de données, est configuré pour :

déterminer un état d'activité en calculant des données statistiques dérivées des mesures inertielles, en fournissant les mesures inertielles et les données statistiques à un modèle prédictif, et en recevant à partir du modèle prédictif un état d'activité déterminé ; et
déterminer le degré de remplissage en utilisant un ou plusieurs parmi (i) un modèle de régression ou (ii) un modèle d'apprentissage automatique pour résoudre une fonction qui présente des entrées indicatives (i) d'un temps écoulé depuis la détermination de la présence d'exsudat corporel déterminée à partir de la mesure normalisée de lumière et (ii) de l'état d'activité ; et
recevoir, à partir du dispositif externe, un degré de remplissage de l'article absorbant.

13. Système selon la revendication 12, dans lequel le dispositif externe est en outre configuré pour calculer un temps jusqu'à ce que l'article absorbant soit censé être rempli en utilisant un modèle de régression pour résoudre une fonction qui présente des entrées indicatives du temps écoulé et de l'état d'activité.

14. Système selon la revendication 12 ou 13, dans lequel le dispositif externe est en outre configuré pour déterminer le degré de remplissage sur la base (iii) d'un temps écoulé supplémentaire depuis que l'article absorbant a été placé sur un nourrisson.

15. Système, comprenant :

un support lisible par ordinateur stockant des instructions exécutables par ordinateur non transitoires ; et
un dispositif de traitement couplé en communication au support lisible par ordinateur pour exécuter les instructions exécutables par ordinateur non transitoires, dans lequel l'exécution des instructions exécutables par ordinateur non transitoires configure le dispositif de traitement pour exécuter le procédé mis en œuvre par ordinateur selon l'une quelconque des revendications 1 à 11.

Color Strip 161

Absorbent
Article 160

100

Color Sensor
120

Movement
Sensor 130

Microcontroller 101

Diaper
Loading
Application
102

Color
Sensing
Application
111

Activity
Classification
Application
116

Data 117

FIG. 1

Ambient Light 250

Object 251

200

Pulsed Light

Light Source 202

Photodetector 204

Processor 206

Transceiver
212

Microcontroller 101

Color
Sensing
Application
111

FIG. 2

Color Strip 351

300

Absorbent Article 301

Sensor Package 310

Rear

Front

FIG. 3

FIG. 4A

FIG. 4B

500

Opaque barrier 510    Light Source 502    Opaque barrier 511

R

G

B

Photodetector 505

Photodetector 515

Color Detector Cell

Separation distance 520

Separation distance 521

FIG. 5

600

601 ┌─────────────────────────────────────────────────────────────────┐
│ Obtain a first measurement of ambient light from a photodetector. │
└─────────────────────────────────────────────────────────────────┘

602 ┌─────────────────────────────────────────────────────────────────┐
│ Cause a light source to transmit light on an object. │
└─────────────────────────────────────────────────────────────────┘

603 ┌─────────────────────────────────────────────────────────────────┐
│ Obtain a second measurement from the photodetector during the transmission, the second measurement including the ambient light and the transmitted light reflected from the object. │
└─────────────────────────────────────────────────────────────────┘

604 ┌─────────────────────────────────────────────────────────────────┐
│ Determine a normalized measurement of the reflected light by removing an ambient light signal from the second measurement based on the first measurement. │
└─────────────────────────────────────────────────────────────────┘

605 ┌─────────────────────────────────────────────────────────────────┐
│ Determine, based on the normalized measurement, one of (i) a presence of bodily exudate or (ii) a volume of bodily exudate present. │
└─────────────────────────────────────────────────────────────────┘

# FIG. 6

700

<div style="border:1px solid">

701 — Receive, from a movement sensor, a time series of data including an inertial measurement for each of a set of time periods.

702 — Calculate, from a subset of the time series data, an activity function such as (i) a variance of the of the inertial measurement or (ii) a root-mean-square of the intertial measurement.

703 — Determine an activity indicated by the subset of time series data based on a measure from the activity function being greater than a first threshold and less than a second threshold.

</div>

# FIG. 7

800

801 → Receive, from a monitoring sensor, a time series of data including an inertial measurement for each of a set of time periods.

802 → Calculate, from the time series data, an activity function such as (i) a variance of the of the inertial measurement or (ii) a root-mean-square of the inertial measurement.

803 → Provide the activity function output such as the (i) variance or (ii) the root mean square of the inertial measurement to a predictive model trained to identify an activity from a list of activities.

804 → Receive, from the predictive model, a determination of an activity corresponding to the subset of time series data.

FIG. 8

900

| |
|---|
| Obtain a first measurement of ambient light received from a photodetector while a light source is off. |

901

↓

| |
|---|
| Obtain a second measurement from the photodetector while the light source is transmitting light. |

902

↓

| |
|---|
| Determine a normalized measurement of light reflected from an absorbent article by removing an ambient light signal from the second measurement based on the first measurement. |

903

↓

| |
|---|
| Determine, from the normalized measurement, a presence of urine in the absorbent article. |

904

↓

| |
|---|
| Determine a degree of fullness in the absorbent article; the determining based (i) an elapsed time since the presence of urine and (ii) an activity state of the infant. |

905

# FIG. 9

1000

Computing System
1000

Memory
1014

| Color sensing application 111 | Diaper Loading Application 115 | Activity Classification Application 116 |

Bus 1006

Network Interface 1004

Processor 1002

I/O Interface 1010

FIG. 10

**EP 3 787 582 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2007128038 A